# EUROPÄISCHE PATENTSCHRIFT

(11) **EP 3 561 040 B1**
(45) Veröffentlichungstag und Bekanntmachung des Hinweises auf die Patenterteilung: **09.09.2020**
(21) Anmeldenummer: 19020304.2
(22) Anmeldetag: 23.04.2019
(51) Int. Cl.: C12M 1/00

(54) **DISPENSIERVORRICHTUNG MIT EINEM DISPENSER ZUM AUSGEBEN EINER WENIGSTENS EINE ZELLE UND/ODER WENIGSTENS EIN PARTIKEL ENTHALTENEN FLÜSSIGKEIT**
DISPENSING MACHINE WITH DISPENSER FOR DISPENSING AT LEAST ONE CELL AND/OR AT LEAST ONE FLUID CONTAINING PARTICLES
DISPOSITIF DE DISTRIBUTION DOTÉ D'UN DISTRIBUTEUR PERMETTANT DE DISTRIBUER UN LIQUIDE CONTENANT AU MOINS UNE CELLULE ET / OU AU MOINS UNE PARTICULE

(30) Priorität: 23.04.2018 LU 100778
(43) Veröffentlichungstag der Anmeldung: 30.10.2019
(73) Patentinhaber: cytena GmbH, 79108 Freiburg (DE)
(72) Erfinder: LAUTSCHAM, Lena, 79108 Freiburg (DE); SCHÖNDUBE, Jonas, 79108 Freiburg (DE)
(74) Vertreter: Dennemeyer & Associates S.A.

(56) Entgegenhaltungen:
- WO-A1-2017/220509
- RIBA J ET AL: "Label-free isolation and deposition of single bacterial cells from heterogeneous samples for clonal culturing", SCIENTIFIC REPORTS, Bd. 6, Nr. 1, 6. September 2016 (2016-09-06), Seiten 1-9, XP055526118, DOI: 10.1038/srep32837
- SHIN D-Y ET AL: "Rapid jetting status inspection and accurate droplet volume measurement for a piezo drop-on-demand inkjet print head using a scanning mirror for display applications", REVIEW OF SCIENTIFIC INSTRUMENTS, AIP, MELVILLE, NY, US, Bd. 88, Nr. 2, 13. Februar 2017 (2017-02-13), Seiten 025109-1-02510913, XP012216056, ISSN: 0034-6748, DOI: 10.1063/1.4975094 [gefunden am 2017-02-13]
- ORTEGA ARROYO J ET AL: "Label-Free, All-Optical Detection, Imaging, and Tracking of a Single Protein", NANO LETTERS, Bd. 14, Nr. 4, 9. April 2014 (2014-04-09), Seiten 2065-2070, XP055269011, US ISSN: 1530-6984, DOI: 10.1021/nl500234t
- DRECHSLER A ET AL: "Confocal microscopy with a high numerical aperture parabolic mirror", OPTICS EXPRESS, Bd. 9, Nr. 12, 3. Dezember 2001 (2001-12-03), Seiten 637-644, XP055526491, US ISSN: 2161-2072, DOI: 10.1364/OE.9.000637
- Gross A ET AL: "Single-Cell Printer: Automated, On Demand, and Label Free", Journal of Laboratory Automation Society for Laboratory Automation and Screening, Bd. 18 1. Dezember 2013 (2013-12-01), Seiten 504-518, XP055276802, Gefunden im Internet: URL:http://jla.sagepub.com/content/18/6/50 4.full.pdf [gefunden am 2016-05-31]

## Beschreibung

Die Erfindung betrifft eine Dispensiervorrichtung mit einem Dispenser zum Ausgeben einer wenigstens eine Zelle und/oder wenigstens ein Partikel enthaltenen Flüssigkeit und einer optischen Erfassungseinrichtung zum optischen Erfassen wenigstens eines Teilbereichs des Dispensers, die eine Lichtquelle zum Aussenden eines Beleuchtungslichts zum Beleuchten des Teilbereichs und eine Umlenkeinrichtung zum Umlenken eines von dem Teilbereich ausgehenden Detektionslichts aufweist.

Aus dem Stand der Technik ist bekannt, dass Wirkstoffe, wie beispielsweise monoklonale Antikörper und andere Proteine mit Hilfe sogenannter monoklonaler Zelllinien hergestellt werden. Dies sind Populationen aus Zellen, die alle von einer einzelnen Mutterzelle abstammen. Das Herstellen von monoklonalen Zelllinien ist notwendig, da nur so sichergestellt werden kann, dass alle Zellen der Population ein annährend gleiches Genom haben, um die Wirkstoffe konstanter und reproduzierbarer Qualität zu erzeugen.

Um eine monoklonale Zelllinie zu erzeugen, werden Zellen einzeln in Behältnisse einer Mikrotiterplatte überführt. Die zu überführenden Zellen werden hergestellt, indem eine Host-Zelllinie genetisch verändert wird und diese veränderten Zellen vereinzelt werden. Das Ablegen einzelner Zellen in die Mikrotiterplatten geschieht durch eine Dispensiervorrichtung. Nach Ablegen der Zellen in die jeweiligen Behältnisse der Mikrotiterplatte können die Zellen wachsen und werden anschließend ggf. in einen Bioreaktor überführt.

Seitens der Benutzer der Dispensiervorrichtung besteht der Wunsch, dass für jede in das Behältnis der Mikrotiterplatte abgelegte Flüssigkeit bekannt ist, ob diese eine Zelle und/oder ein Partikel enthält. Die Dispensiervorrichtung weist daher üblicherweise eine optische Erfassungseinrichtung auf, mittels der ermittelt wird, ob die ausgegebene Flüssigkeit eine Zelle und/oder ein Partikel enthält.

Um Abbildungen mit einer hohen Auflösung zu erhalten, ist es empfehlenswert, dass die optische Erfassungseinrichtung möglichst nah an einem Dispenser der Dispensiervorrichtung platziert wird. Dies ist jedoch aufgrund der konstruktiven Gegebenheiten nicht möglich. So soll der Abstand zwischen dem Dispenser und dem Behältnis zum Aufnehmen der ausgegebenen Flüssigkeit möglichst gering sein, damit sichergestellt ist, dass die ausgegebene Flüssigkeit auch tatsächlich in dem Behältnis landet. Dies führt jedoch dazu, dass die optische Erfassungsvorrichtung nicht nah an den Dispenser angeordnet werden kann und somit komplex ausgebildete optische Erfassungsvorrichtungen verwendet werden müssen.

RIBA J ET AL: "Label-free isolation and deposition of single bacterial cells from heterogeneous samples for clonal culturing", SCIENTIFIC REPORTS, Bd. 6, Nr. 1, 06. September 2016, offenbart eine Vorrichtung zum Ausgeben einer Flüssigkeit, die bakterielle Zellen enthalten kann. Die Vorrichtung weist eine optische Erfassungseinrichtung mit einem Objektiv und einem Umlenkspiegel auf.

SHIN D-Y ET AL: "Rapid jetting status inspection and accurate droplet volume measurement for a piezo drop-on-demand inkjet print head using a scanning mirror for display applications", REVIEW OF SCIENTIFIC INSTRUMENTS, AIP, MELVILLE, NY, US, Bd. 88, Nr. 2, 13. Februar 2017, offenbart einen Tintenstrahldruckkopf, der einen drehbaren Spiegel aufweist.

ORTEGA ARROYO J ET AL: "Label-Free, All-Optical Detection, Imaging, and Tracking of a Single Protein", NANO LETTERS, Bd. 14, Nr. 4, 9. April 2014 offenbart eine Vorrichtung zum Erfassen, Aufnehmen und Verfolgen eines einzelnen Proteins. Die Vorrichtung weist einen polarisierenden Strahlteiler auf, der Detektionslicht zu einer CMOS Kamera umlenkt.

DRECHSLER A ET AL: "Confocal microscopy with a high numerical aperture parabolic mirror", OPTICS EXPRESS, Bd. 9, Nr. 12, 3. Dezember 2001, offenbart ein Konfokalmikroskop mit einem Parabolspiegel.

Gross A ET AL: "Single-Ceii Printer: Automated, On Demand, and Labei Free", Journai of Laboratory Automation Society for Laboratory Automation and Screening, Bd. 18, 1. Dezember 2013 offenbart eine Vorrichtung zum Ausgeben einer Flüssigkeit. Die Vorrichtung weist eine Aufnahmevorrichtung zum Aufnehmen der ausgegebenen Flüssigkeit auf.

WO 2017/ 220 509 A1 offenbart eine Vorrichtung zum Erkennen von Zellen oder Partikeln in einem Fluidbehälter. Die Vorrichtung umfasst einen Dispenser der zum Dispensieren von mindestens einer Zelle oder mindestens einem Partikel dient. Darüber hinaus weist die Vorrichtung eine Erfassungsvorrichtung zum Erfassen der Zelle oder des Partikels auf.

Eine Aufgabe der Erfindung besteht daher darin, eine verbesserte Dispensiervorrichtung anzugeben.

Die Aufgabe wird durch eine Dispensiervorrichtung der eingangs genannten Art gelöst, die dadurch gekennzeichnet ist, dass die Umlenkeinrichtung das Detektionslicht wenigstens zweimal, insbesondere genau zweimal, umlenkt, wobei die Umlenkeinrichtung wenigstens ein Prisma zum Umlenken des Detektionslichts und/oder des Beleuchtungslichts aufweist.

Die erfindungsgemäße Vorrichtung weist den Vorteil auf, dass auch bei Verwenden eines Standardobjektivs eine hohe Auflösung und/oder eine hohe Vergrößerung des betrachteten Teilbereichs realisiert werden kann. Dies ist möglich, weil durch die Umlenkeinrichtung eine höhere numerische Apertur genutzt werden kann. Insbesondere ist es in der erfindungsgemäßen Vorrichtung nicht notwendig, teure Objektive einzusetzen, sondern es kann ein kostengünstigeres Standardobjektiv eingesetzt werden. Das Vorsehen der Umlenkeinrichtung bietet außerdem den Vorteil, dass die nachfolgend näher beschriebenen Bestandteile der optischen Erfassungseinrichtung zum optischen Erfassen des Teilbereichs des Dispensers nicht bewegt, insbesondere nicht relativ zu dem Dispenser bewegt, werden müssen.

Ein weiterer Vorteil der Erfindung besteht darin, dass ein Teilbereich des Dispensers mit dem Beleuchtungslicht beleuchtet wird. Somit kann durch Betrachten des Teilbereichs des Dispensers ermittelt werden, ob die auszugebende Flüssigkeit eine Zelle oder ein Partikel enthält. Vorzugsweise entspricht der Teilbereich des Dispensers einem Ausgabebereich des Dispensers, insbesondere einem eine Düse aufweisenden Ausgabebereich des Dispensers. Somit ist es nicht notwendig, die aus dem Dispenser tatsächlich ausgegebene Flüssigkeit zu betrachten. Das Betrachten des Teilbereichs des Dispensers bietet den Vorteil, dass keine optischen Elemente zwischen dem Dispenser und einer Mikrotiterplatte angeordnet werden müssen. Daher kann der Abstand zwischen dem Dispenser und der Mikrotiterplatte klein sein.

Mittels des Dispensers wird die Flüssigkeit, insbesondere ein Flüssigkeitstropfen, ausgegeben. Dabei kann die ausgegebene Flüssigkeit keine Zelle und/oder kein Partikel enthalten. Alternativ kann die ausgegebene Flüssigkeit genau eine einzige Zelle oder mehrere Zellen und/oder ein einziges Partikel oder mehrere Partikel enthalten.

Der Dispenser kann mit einem Flüssigkeitsreservoir der Dispensiervorrichtung fluidisch verbunden sein. Dabei kann der Flüssigkeitstropfen wenigstens eine Zelle und/oder wenigstens ein Partikel aufweisen. Die ausgegebene Flüssigkeit weist ein Volumen in einem Bereich zwischen 10pl (Pikoliter) bis 50 nl (Nanoliter) auf. Die Flüssigkeit kann eine Zellsuspension sein, die das Wachstum der Zellen fördert. Das Partikel kann ein Glas- oder Polymerkügelchen sein und im Wesentlichen das gleiche Volumen wie die Zelle aufweisen. Die Lichtquelle kann eine LED-Lampe sein.

Das infolge des Beleuchtens des Teilbereichs mit dem Beleuchtungslicht von dem Teilbereich ausgehende Detektionslicht kann detektiert werden. Das detektierte Detektionslicht kann mittels einer nachstehend näher beschriebenen Ausleseeinrichtung ausgelesen und anschließend ausgewertet werden.

Bei einer besonderen Ausführung kann die Umlenkeinrichtung das Beleuchtungslicht wenigstens zweimal, insbesondere genau zweimal, umlenken. Dies bedeutet, dass die Umlenkeinrichtung derart ausgebildet und angeordnet ist, dass sie sowohl das Detektionslicht als auch das Beleuchtungslicht umlenkt. Dadurch kann eine kompakt ausgebildete optische Erfassungseinrichtung realisiert werden. Dabei kann das Detektionslicht bei jedem Umlenkvorgang im Wesentlichen um 90°, insbesondere genau um 90°, umgelenkt werden. Analog kann das Beleuchtungslicht bei jedem Umlenkvorgang im Wesentlichen um 90°, insbesondere jeweils genau um 90°, umgelenkt werden.

Darüber hinaus kann die Umlenkeinrichtung derart ausgebildet sein, dass das durch eine erste Seite der Umlenkeinrichtung austretende Beleuchtungslicht zu dem durch eine zweite Seite eintretenden Beleuchtungslicht wenigstens zweimal umgelenkt ist. Dabei kann die Umlenkeinrichtung derart ausgebildet sein, dass das aus der Umlenkeinrichtung austretende Beleuchtungslicht versetzt zu dem in die Umlenkeinrichtung eintretenden Beleuchtungslicht verläuft. Insbesondere kann die Umlenkeinrichtung das aus der Umlenkeinrichtung austretende Beleuchtungslicht im Wesentlichen parallel, insbesondere parallel, zu dem in die Umlenkeinrichtung eintretenden Beleuchtungslicht verschieben. Die Umlenkung des Beleuchtungslichts kann analog zu dem Detektionslicht bei jedem Umlenkvorgang im Wesentlichen um 90°, insbesondere genau um 90°, erfolgen.

Dabei kann das aus der Umlenkeinrichtung austretende Detektionslicht versetzt zu dem in die Umlenkeinrichtung eintretenden Detektionslicht verlaufen. Besonders vorteilhaft ist, wenn das aus der Umlenkeinrichtung austretende Detektionslicht im Wesentlichen parallel, insbesondere parallel, zu dem in die Umlenkeinrichtung eintretenden Detektionslicht verschoben ist. Außerdem kann die Umlenkeinrichtung derart ausgebildet sein, dass das durch die zweite Seite der Umlenkeinrichtung austretende Detektionslicht zu dem durch die erste Seite eintretenden Detektionslicht wenigstens zweimal umgelenkt ist.

Das Prisma bietet den Vorteil, dass das aus dem Prisma austretende Beleuchtungslicht auf einfache Weise zu dem in das Prisma eintretende Beleuchtungslicht versetzt, insbesondere parallel verschoben, werden kann. Gleichermaßen kann das aus dem Prisma austretende Detektionslicht auf einfache Weise zu dem in das Prisma eintretende Detektionslicht versetzt, insbesondere parallel verschoben, werden. Die Verschiebung erfolgt besonders einfach, wenn das Prisma eine Grundfläche mit wenigstens vier, insbesondere genau vier, Ecken aufweist. Dabei kann das Prisma ein schiefes Prisma, insbesondere ein Parallelepiped sein. Vorzugsweise kann das Prisma ein Rhomboeder sein. Das Verwenden eines Prismas weist den Vorteil auf, dass eine genaue parallele Verschiebung des Beleuchtungslichts und/oder des Detektionslichts realisiert werden kann.

Bei Verwenden des Prismas entspricht die erste Seite einer Mantelfläche des Prismas und die zweite Seite entspricht einer anderen Mantelfläche des Prismas. Die erste Seite, insbesondere die Mantelfläche, und die zweite Seite, insbesondere die andere Mantelfläche, können parallel zueinander verlaufen.

Alternativ oder zusätzlich kann die Umlenkeinrichtung wenigstens zwei Spiegel zum Umlenken des Beleuchtungslichts und/oder des Detektionslichts aufweisen. Unabhängig davon, ob die Umlenkeinrichtung das Prisma oder die Spiegel aufweist, ist die Umlenkeinrichtung in beiden Fällen derart ausgebildet, dass sie das Beleuchtungslicht in Richtung zum Teilbereich des Dispensers umlenkt, damit der Teilbereich des Dispensers durch das Beleuchtungslicht beleuchtet wird.

Die optische Erfassungseinrichtung kann ein Objektiv aufweisen. Das Objektiv ist ein sammelndes optisches System, das eine reelle optische Abbildung des Gegenstands erzeugt. Die Umlenkeinrichtung kann im Strahlengang des Beleuchtungslichts und/oder Detektionslichts zwischen dem Objektiv und dem Dispenser angeordnet ist. Dies ermöglicht, dass die Umlenkeinrichtung mit dem Dispenser mechanisch verbunden sein kann, wodurch eine kompakte Vorrichtung realisiert werden kann. Insbesondere kann die Umlenkeinrichtung, insbesondere direkt, an dem Dispenser angebracht sein. Dadurch wird ermöglicht, dass der Abstand zwischen dem Objektiv und dem Dispenser klein ist, was wiederum für die Auflösung und die Vergrößerung von Vorteil ist.

Die Umlenkeinrichtung kann das Beleuchtungslicht direkt in den Teilbereich des Dispensers umlenken. Des bedeutet, dass im Strahlengang des Beleuchtungslichts keine weiteren optischen Elemente zwischen dem Dispenser und der Umlenkeinrichtung angeordnet sind. Darüber hinaus kann die Umlenkeinrichtung das Detektionslicht direkt in das Objektiv umlenken. Dies bedeutet, dass im Strahlengang des Detektionslichts keine weiteren optischen Elemente zwischen dem Objektiv und der Umlenkeinrichtung angeordnet sind.

Wenigstens eine Linse kann an der zum Dispenser zugewandten Seite der Umlenkeinrichtung, insbesondere dem Prisma, angebracht oder angeordnet sein. Dabei kann die Linse eine plane Seite aufweisen, die an der zum Dispenser zugewandten Seite der Umlenkeinrichtung, insbesondere der Lichtaustrittsseite, angebracht ist. Dabei kann die plane Seite der Linse an einer Mantelfläche des Prismas, vorzugsweise direkt, angebracht sein. Durch Verwenden der Linse können Abbildungsfehler, die insbesondere aus einer sphärischen Aberration resultieren, auf einfache Weise zumindest teilweise behoben werden.

Das Objektiv kann derart angeordnet sein, dass eine optische Achse des Objektivs quer, insbesondere senkrecht, zu einer Ausgaberichtung der Flüssigkeit aus dem Dispenser verläuft. Eine derartige Anordnung des Objektivs bietet den Vorteil, dass das Objektiv näher an dem Dispenser angeordnet werden kann als bei den bekannten Dispensiervorrichtungen, bei denen das Objektiv oberhalb des Dispensers angeordnet ist. Im Ergebnis erhöht sich durch die zuvor beschriebene Anordnung des Objektivs die Auflösung und/oder die Vergrößerung.

Die Dispensiervorrichtung kann einen Aktor zum Betätigen des Dispensers aufweisen. Der Aktor kann ein Piezoaktor sein. Das Objektiv und der Aktor können sich bezogen auf den Dispenser gegenüberliegen. Insbesondere können sich der Aktor und das Objektiv bezogen auf eine Ebene, die in die gleiche Richtung verläuft wie eine Ausbringrichtung der Flüssigkeit aus dem Dispenser, gegenüberliegen.

Bei einer besonderen Ausführung kann die optische Erfassungseinrichtung eine Ausleseeinrichtung zum Auslesen des von dem Teilbereich des Dispensers ausgehenden detektierten Detektionslichts aufweisen. Die Ausleseeinrichtung dient zum Auslesen der in dem detektierten Detektionslicht enthaltenen Informationen. Das Detektionslicht resultiert aus dem Beleuchten des Teilbereichs mit dem Beleuchtungslicht und/oder weist beispielsweise an den Zellen und/oder Partikeln reflektiertes Beleuchtungslicht auf.

Das Detektionslicht und das Beleuchtungslicht können wenigstens teilweise einen gemeinsamen Strahlengang besitzen. Der gemeinsame Strahlengang kann wenigstens das Objektiv und die Umlenkeinrichtung aufweisen.

Darüber hinaus kann die optische Erfassungseinrichtung eine Abbildungsvorrichtung aufweisen. Die Abbildungsvorrichtung kann basierend auf dem detektierten Detektionslicht eine Abbildung erzeugen. Insbesondere kann mittels der Abbildungsvorrichtung eine Abbildung von dem Teilbereich des Dispensers erzeugt werden. Der Teilbereich des Dispensers kann den Ausgabebereich des Dispensers, insbesondere die Düse des Dispensers, aufweisen. Die Ausleseeinrichtung kann in der Abbildungsvorrichtung integriert sein. Die Abbildungsvorrichtung kann eine Kamera sein.

Die optische Erfassungseinrichtung kann außerdem eine Auswerteeinrichtung zum Auswerten des detektierten Detektionslichts aufweisen. Dabei kann die Auswerteeinrichtung das detektierte Detektionslicht auswerten, um eine optische Eigenschaft des Teilbereichs zu ermitteln. Insbesondere kann die Auswerteeinrichtung die mittels der Ausleseeinrichtung ausgelesenen Informationen auswerten. Dabei kann beispielsweise eine Transparenz und/oder ein Kontrast und/oder Reflexionsgrad des Teilbereichs als optische Eigenschaft ermittelt werden. Alternativ oder zusätzlich können auch noch weitere optische Eigenschaften des Teilbereichs ermittelt werden.

Die Auswerteeinrichtung kann basierend auf dem detektierten Detektionslicht, insbesondere basierend auf der bestimmten optischen Eigenschaft des Teilbereichs, bestimmen, ob im Teilbereich des Dispensers keine Zelle und/oder kein Partikel angeordnet ist oder ob im Teilbereich des Dispensers eine einzige Zelle und/oder ein einziges Partikel angeordnet ist oder mehrere Zellen und/oder mehrere Partikel angeordnet ist. Im Ergebnis kann auf einfache Weise durch die Auswerteeinrichtung die Anzahl der im Teilbereich angeordneten Zellen und/oder Partikel bestimmt werden. Die Auswerteeinrichtung kann in der Abbildungsvorrichtung integriert sein.

In der Auswerteeinrichtung kann ein Algorithmus zum wenigstens teilweisen Beheben von einem Abbildungsfehler, der beispielsweise aus der sphärischen Aberration resultiert, hinterlegt sein. Dadurch lassen sich durch die Abbildungsvorrichtung Abbildungen mit einer besseren Qualität erzeugen.

Bei einer besonderen Ausführung kann die optische Erfassungseinrichtung eine weitere Lichtquelle zum Ausgeben eines Anregungslichts zum Beleuchten des wenigstens einen Teilbereichs des Dispensers und/oder wenigstens eines anderen Teilbereichs des Dispensers aufweist. Die weitere Lichtquelle kann ein Laser sein. Eine ganz besondere Ausführung wird erhalten, wenn das Anregungslicht und das Beleuchtungslicht wenigstens teilweise einen gemeinsamen Strahlengang besitzen. Bei dieser Ausführung wird ein einfach und kompakt aufgebautes optisches Erfassungssystem erhalten. Der gemeinsame Strahlengang kann wenigstens das Objektiv und die Umlenkeinrichtung aufweisen.

Die Ausleseeinrichtung kann derart angeordnet und ausgebildet sein, dass sie ein von dem Teilbereich des Dispensers ausgehendes detektiertes weiteres Detektionslichts ausliest. Das weitere Detektionslicht resultiert aus dem Beleuchten des Teilbereichs und/oder des anderen Teilbereichs des Dispensers. Das weitere Detektionslicht kann analog zu dem Detektionslicht durch die Umlenkeinrichtung zweimal umgelenkt werden. Insbesondere kann das aus der Umlenkeinrichtung austretende weitere Detektionslicht im Wesentlichen parallel, insbesondere parallel, zu dem in die Umlenkeinrichtung eintretenden weiteren Detektionslicht verschoben sein.

Die Abbildungsvorrichtung kann basierend auf dem detektierten weiteren Detektionslicht eine weitere Abbildung des Teilbereichs und/oder des anderen Teilbereichs erzeugen. Darüber hinaus kann die Auswerteeinrichtung zum Bestimmen einer weiteren optischen Eigenschaft des Teilbereichs und/oder des anderen Teilbereichs das weitere Detektionslicht auswerten. Die weitere optische Eigenschaft kann ein Fluoreszieren der in dem Teilbereich angeordneten Zelle und/oder des im Teilbereich angeordneten Partikels sein. In diesem Fall dient das Anregungslicht zum Anregen der Zelle und/oder des Partikel. Dabei wird das Anregungslicht durch eine fluoreszierende Substanz der Zelle und/oder eine fluoreszierende Substanz des Partikels absorbiert. Das von der Zelle und/oder dem Partikel emittierte weitere Detektionslicht entspricht einem Fluoreszenzlicht. Dabei kann die Auswerteeinrichtung basierend auf dem weiteren Detektionslicht ermitteln, ob im Teilbereich und/oder anderen Teilbereich keine fluoreszierende Zelle und/oder kein fluoreszierendes Partikel angeordnet ist oder ob im Teilbereich und/oder anderen Teilbereich genau eine einzige fluoreszierende Zelle und/oder genau ein einziges fluoreszierendes Partikel angeordnet ist und/oder ob im Teilbereich mehrere Zellen und/oder mehrere Partikel angeordnet sind.

Das weitere Detektionslicht und das Anregungslicht können wenigstens teilweise einen gemeinsamen Strahlengang besitzen. Der gemeinsame Strahlengang kann wenigstens das Objektiv und die Umlenkeinrichtung aufweisen.

Die Verwendung der Ausleseeinrichtung zum Auslesen des detektierten Detektionslichts und des detektierten weiteren Detektionslichts und der Abbildungsvorrichtung zum Erzeugen der Abbildung und der weiteren Abbildung und der Auswerteeinrichtung zum Bestimmen der weiteren optischen Eigenschaft bietet den Vorteil, dass ein optisches Erfassungssystem mit wenigen Elementen realisiert wird.

Das Vorsehen der Umlenkeinrichtung ist insbesondere bei der Ausführung vorteilhaft, bei der ermittelt wird, ob im Teilbereich eine fluoreszierende Zelle und/oder ein fluoreszierendes Partikel angeordnet ist. Wie oben bereits beschrieben ist, bietet das Vorsehen der Umlenkeinrichtung den Vorteil einer höheren numerischen Apertur. Dies bedeutet, dass aufgrund des daraus resultierenden größeren Abstrahlwinkels ein größerer Bereich abgedeckt ist, in dem die von der Zelle und/oder dem Partikel ausgehenden Photonen zum Objektiv gelangen können. Insofern kann aufgrund der Umlenkeinrichtung auch eine bessere Abbildung des Teilbereichs erhalten werden. Außerdem können aufgrund des größeren Abstrahlwinkels die Zelle und/oder das Partikel kürzer angeregt werden, was eine schnellere Flüssigkeitsausgabe aus dem Dispenser ermöglicht.

Bei einer alternativen Ausführung kann die optische Erfassungseinrichtung eine weitere Ausleseeinrichtung zum Auslesen eines detektierten weiteren Detektionslichts aufweisen. Darüber hinaus kann die optische Erfassungseinrichtung eine weitere Abbildungsvorrichtung zum Erzeugen einer weiteren Abbildung des Teilbereichs und/oder des anderen Teilbereichs basierend auf dem detektierten weiteren Detektionslicht und/oder eine weitere Auswerteeinrichtung aufweisen. Die weitere Auswerteeinrichtung kann zum Bestimmen der weiteren optischen Eigenschaft des Teilbereichs das weitere Detektionslicht auswerten. Dabei kann die weitere Auswerteeinrichtung basierend auf dem weiteren Detektionslicht bestimmen, ob im Teilbereich und/oder anderen Teilbereich keine fluoreszierende Zelle und/oder kein fluoreszierendes Partikel angeordnet ist oder ob im Teilbereich und/oder anderen Teilbereich genau eine einzige fluoreszierende Zelle und/oder genau ein einziges fluoreszierendes Partikel angeordnet ist und/oder ob im Teilbereich mehrere Zellen und/oder mehrere Partikel angeordnet sind.. Die weitere Abbildungsvorrichtung kann eine Kamera sein und/oder die weitere Auswerteeinrichtung und/oder die weitere Ausleseeinrichtung können in der Abbildungsvorrichtung integriert sein.

Die Dispensiervorrichtung kann eine Ablenk- und/oder Absaugeinrichtung aufweisen. Die Ablenkeinrichtung dient zum Ablenken der ausgegebenen Flüssigkeit, insbesondere des ausgegebenen Flüssigkeitstropfens. Die Absaugeinrichtung dient zum Absaugen der ausgegebenen Flüssigkeit. Die ausgegebene Flüssigkeit kann in ein Ausschussbehältnis abgelenkt und/oder abgesaugt werden. Das Ablenken und/oder Absaugen kann erfolgen, bevor die ausgegebene Flüssigkeit in das Behältnis, insbesondere das Behältnis einer Mikrotiterplatte eintritt. Dabei kann die ausgegebene Flüssigkeit abgelenkt und/oder abgesaugt werden, wenn die Flüssigkeit keine Zellen und/oder keine Partikel enthält. Alternativ kann die ausgegebene Flüssigkeit abgelenkt und/oder abgesaugt werden, wenn die Anzahl der in der Flüssigkeit enthaltenen Zellen und/oder Partikel größer als ein vorgegebener Wert, insbesondere größer als 1 ist.

In den Figuren ist der Erfindungsgegenstand schematisch dargestellt, wobei gleiche oder gleichwirkende Elemente zumeist mit denselben Bezugszeichen versehen sind. Dabei zeigt:
Figur 1 eine schematische Darstellung einer erfindungsgemäßen Dispensiervorrichtung gemäß einer ersten Ausführung.
Figur 2 eine schematische Darstellung einer erfindungsgemäßen Dispensiervorrichtung gemäß einer zweiten Ausführung.

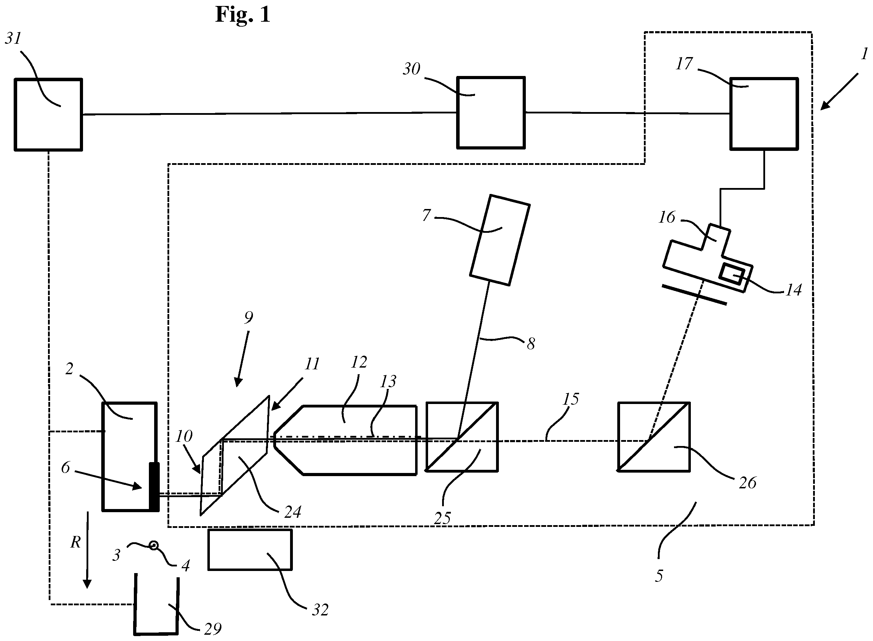

Figur 1 zeigt eine Dispensiervorrichtung 1, die einen Dispenser 2 zum Ausgeben einer wenigstens eine Zelle 3 und/oder wenigstens ein Partikel enthaltenen Flüssigkeit 4 und eine optische Erfassungseinrichtung 5 zum optischen Erfassen wenigstens eines Teilbereichs 6 des Dispensers 2 aufweist. Die optische Erfassungseinrichtung 5 weist eine Lichtquelle 7, wie beispielsweise eine LED-Lampe, zum Aussenden eines Beleuchtungslichts 8 und eine ein Prisma 24 aufweisende Umlenkeinrichtung 9 auf. Das Beleuchtungslicht 8 dient zum Beleuchten des Teilbereichs 6 und wird von der Umlenkeinrichtung 9 zweimal umgelenkt. Ein daraufhin von dem Teilbereich 6 ausgehendes Detektionslicht 15 wird von der Umlenkeinrichtung 9 ebenfalls zweimal umgelenkt.

Die Umlenkeinrichtung 9 ist derart ausgebildet, dass das durch die erste Seite 10 des Prismas austretende Beleuchtungslicht 8 zu dem durch die zweite Seite 11 des Prismas eintretenden Beleuchtungslicht 8 parallel verschoben ist. Gleichermaßen ist das durch die zweite Seite 11 des Prismas austretende Detektionslicht 15 zu dem durch die erste Seite 10 des Prismas eintretenden Detektionslicht 15 parallel verschoben. Figur 1 zeigt einen Zustand, bei dem die Lichtquelle 7 das Beleuchtungslicht 8 aussendet.

Die Umlenkeinrichtung 9 ist derart angeordnet, dass sie das Beleuchtungslicht 8 zum Teilbereich 6 des Dispensers 2 umlenkt. Der Teilbereich 6 des Dispensers 2 entspricht einem Ausgabebereich des Dispensers 2. In Figur 1 ist ein Zustand gezeigt, bei dem der Dispenser 2 eine Flüssigkeit 4, insbesondere einen Flüssigkeitstropfen, ausgegeben hat, der eine Zelle 3 enthält. Die Flüssigkeit 4 wird in ein Behältnis 29 zugeführt. Der Dispenser 2 wird durch einen nicht dargestellten Aktuator, insbesondere Piezoaktor, betätigt.

Die optische Erfassungseinrichtung 5 weist außerdem ein Objektiv 12 auf.

Das Objektiv 12 ist derart angeordnet, dass eine optische Achse 13 des Objektivs 12 senkrecht zu einer Ausgaberichtung R der Flüssigkeit 4 aus dem Dispenser 2 verläuft. Die Umlenkeinrichtung 9 ist im Strahlengang des Beleuchtungslichts 8 und/oder des Detektionslichts 15 zwischen dem Dispenser 2 und dem Objektiv 12 angeordnet.

Darüber hinaus weist die optische Erfassungseinrichtung 5 einen Filter 25 auf, der im Strahlengang des Beleuchtungslichts 8 zwischen dem Objektiv 12 und der Lichtquelle 7 angeordnet ist. Der Filter 25 ist derart ausgebildet, dass er das Beleuchtungslicht 8 in Richtung zum Objektiv 12 umlenkt. Darüber hinaus ist der Filter 25 derart ausgebildet, dass er das von dem Teilbereich 6 ausgehende Detektionslicht 15 das in Figur 1 gestrichelt dargestellt ist, durchlässt. Das von dem Filter 25 durchgelassene Detektionslicht 15 wird durch einen Strahlumlenker 26 in Richtung zu einer Abbildungsvorrichtung 16 umgelenkt. Das Detektionslicht 15 und das Beleuchtungslicht 8 weisen teilweise einen gemeinsamen Strahlengang auf.

Eine in der Abbildungsvorrichtung 16 angeordnete Ausleseeinrichtung 14 liest das durch einen Detektor der Abbildungsvorrichtung 16 detektierte Detektionslicht 15 aus. Die Abbildungsvorrichtung 16 kann basierend auf dem detektierten Detektionslicht 15, insbesondere auf den durch die Ausleseeinrichtung 14 ausgelesenen Informationen, eine Abbildung des Teilbereichs 6 erzeugen.

Die Abbildungsvorrichtung 16 ist mit einer Auswerteeinrichtung 17, die beispielsweise ein Rechner ist, der optischen Erfassungseinrichtung 5 elektrisch verbunden. Die Auswerteeinrichtung 17 kann das erfasste Detektionslicht 15 auswerten. Insbesondere kann die Auswerteeinrichtung 17 die mittels der Ausleseeinrichtung 14 ausgelesenen Informationen auswerten. Dabei kann die Auswerteeinrichtung 17 basierend auf dem Detektionslicht 15 eine optische Eigenschaft des Teilbereichs 6 bestimmen.

Durch Bestimmen der optischen Eigenschaft des Teilbereichs 6 ist es möglich, zu bestimmen, ob in dem beleuchteten Teilbereich keine Zelle 3 und/oder kein Partikel angeordnet ist oder wenigstens eine Zelle 3 und/oder wenigstens ein Partikel angeordnet ist

Die Auswerteeinrichtung 17 ist mit einer Steuervorrichtung 28 elektrisch verbunden. Die Steuervorrichtung 30 steuert basierend auf dem Auswerteergebnis der Auswerteeinrichtung 17 den Dispensiervorgang des Dispensers 2. Dabei ist die Steuervorrichtung 30 mit einer Verfahreinrichtung 29 elektrisch verbunden. Die Verfahreinrichtung 29 kann den Dispenser 2 und/oder das Behältnis 29 derart verfahren, dass die Flüssigkeit 4 in den gewünschten Ablageort abgegeben werden kann.

Darüber hinaus kann die Steuervorrichtung 30 eine Ablenk- und/oder Absaugeinrichtung 32 der Dispensiereinrichtung 1 steuern. Dabei kann die Steuervorrichtung 30 die Ablenk- und/oder Absaugeinrichtung 32 derart steuern, dass die ausgegebene Flüssigkeit 4 abgelenkt und/oder abgesaugt wird, wenn in der Flüssigkeit 4 keine Zelle 3 und/oder kein Partikel angeordnet ist oder wenn in der Flüssigkeit 4 mehrere Zellen 3 und/oder mehrere Partikel angeordnet sind.

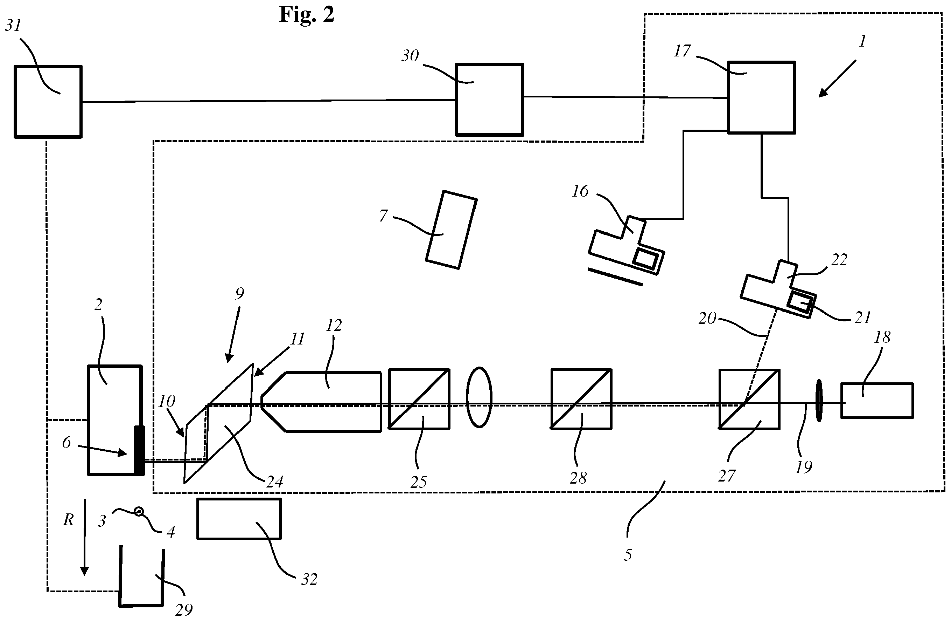

Figur 2 eine schematische Darstellung einer erfindungsgemäßen Dispensiervorrichtung gemäß einer zweiten Ausführung. Die zweite Ausführung unterscheidet sich von der in Figur 1 dargestellten ersten Ausführung darin, dass die optische Erfassungseinrichtung 5 zusätzlich eine weitere Lichtquelle 18, eine weitere Abbildungsvorrichtung 22 und eine weitere Ausleseeinrichtung 21 aufweist. Die weitere Lichtquelle 18 kann ein Laser sein und die weitere Abbildungsvorrichtung 22 kann eine Kamera sein. Figur 2 zeigt einen Zustand, bei dem die weitere Lichtquelle 18 ein Anregungslicht 19 aussendet. Die Lichtquelle 7 sendet dagegen kein Beleuchtungslicht 8 aus.

Ein weiterer Unterschied besteht darin, dass die zweite Ausführung anstelle des Strahlumlenkers 26 einen weiteren Filter 28 aufweist. Der weitere Filter 28 ist derart ausgebildet, dass er das nicht dargestellte Detektionslicht 15 zur Abbildungsvorrichtung 16 umlenkt. Darüber hinaus ist der weitere Filter 28 derart ausgebildet, dass er ein weiteres Detektionslicht 20 durchlässt. Die optische Erfassungseinrichtung 5 weist außerdem einen weiteren Strahlumlenker 27 auf, der das weitere Detektionslicht 20 in Richtung zur weiteren Abbildungsvorrichtung 22 umlenkt, wobei das weitere Detektionslicht 20 durch einen Detektor der weiteren Abbildungsvorrichtung 22 detektiert wird. Das weitere Detektionslicht 20 wird durch die Umlenkeinrichtung 9 analog zu dem Detektionslicht 15 zweimal umgelenkt.

Die weitere Lichtquelle 18 sendet das Anregungslicht 19 zum Beleuchten des Teilbereichs 6 des Dispensers 2 aus, das durch den weiteren Strahlumlenker 27, den weiteren Filter 28, den Filter 25 zum Objektiv 12 durchgeleitet wird. Dabei weist das Anregungslicht 19 und das Beleuchtungslicht 8 einen gemeinsamen Strahlengang auf. Insbesondere wird sowohl das Beleuchtungslicht 8 als auch das Anregungslicht 19 durch das Objektiv 12 und die Umlenkeinrichtung 9 durchgeleitet.

Das von dem Teilbereich 6 infolge des Anregungslichts 19 ausgehende detektierte weitere Detektionslicht 20 wird durch die weitere Ausleseeinrichtung 21 ausgelesen. Bei dem weiteren Detektionslicht 20 kann es sich um von der Zelle 3 und/oder von dem Partikel emittiertes Fluoreszenzlicht handeln, sofern eine Zelle 3 und/oder ein Partikel mit Fluoreszenzeigenschaft in dem Teilbereich 6 des Dispensers 2 angeordnet ist. Die weitere Abbildungsvorrichtung 22 kann basierend auf dem weiteren Detektionslicht 20 eine weitere Abbildung des Teilbereichs 6 des Dispensers 2 erzeugen. Die weitere Auswerteeinrichtung 23 wertet das weitere Detektionslicht 20 aus und ermittelt, ob in dem Teilbereich 6 keine fluoreszierende Zelle 3 und/oder kein fluoreszierendes Partikel angeordnet ist oder ob in dem Teilbereich 6 wenigstens eine Zelle 3 und/oder wenigstens ein Partikel angeordnet ist.

Das Anregungslicht 19 und das weitere Detektionslicht 20 weisen teilweise einen gemeinsamen Strahlengang auf.

### Bezugszeichenliste:

- 1: Dispensiervorrichtung
- 2: Dispenser
- 3: Zelle
- 4: Flüssigkeit
- 5: optische Erfassungseinrichtung
- 6: Teilbereich
- 7: Lichtquelle
- 8: Beleuchtungslicht
- 9: Umlenkeinrichtung
- 10: erste Seite
- 11: zweite Seite
- 12: Objektiv
- 13: optische Achse
- 14: Ausleseeinrichtung
- 15: Detektionslicht
- 16: Abbildungsvorrichtung
- 17: Auswerteeinrichtung
- 18: weitere Lichtquelle
- 19: Anregungslicht
- 20: weiteres Detektionslicht
- 21: weitere Ausleseeinrichtung
- 22: weitere Abbildungsvorrichtung
- 23: weitere Auswerteeinrichtung
- 24: Prisma
- 25: Filter
- 26: Strahlumlenker
- 27: weiterer Strahlumlenker
- 28: weiterer Filter
- 29: Behältnis
- 30: Steuervorrichtung
- 31: Verfahreinrichtung
- 32: Ablenk- und/oder Absaugeinrichtung

- R: Ausgaberichtung

## Patentansprüche

1. Dispensiervorrichtung (1) mit einem Dispenser (2) zum Ausgeben einer wenigstens eine Zelle (3) und/oder wenigstens ein Partikel enthaltenen Flüssigkeit (4) und einer optischen Erfassungseinrichtung (5) zum optischen Erfassen wenigstens eines Teilbereichs (6) des Dispensers (2), die eine Lichtquelle (7) zum Aussenden eines Beleuchtungslichts (8) zum Beleuchten des Teilbereichs (6) und eine Umlenkeinrichtung (9) zum Umlenken eines von dem Teilbereich (6) ausgehenden Detektionslichts (15) aufweist, **dadurch gekennzeichnet, dass** die Umlenkeinrichtung (9) das Detektionslicht (15) wenigstens zweimal, insbesondere genau zweimal, umlenkt, wobei die Umlenkeinrichtung (9) wenigstens ein Prisma (24) zum Umlenken des Detektionslichts (15) und/oder des Beleuchtungslichts (8) aufweist.

2. Dispensiervorrichtung (1) nach Anspruch 1, **dadurch gekennzeichnet, dass**
a. die Umlenkeinrichtung (9) das Beleuchtungslicht (8) wenigstens zweimal, insbesondere genau zweimal, umlenkt und/oder dass
b. die Umlenkeinrichtung (9) derart ausgebildet ist, dass das durch eine erste Seite (10) der Umlenkeinrichtung austretende Beleuchtungslicht (8) zu dem durch eine zweite Seite (11) eintretenden Beleuchtungslicht (8) wenigstens zweimal umgelenkt ist und/oder dass
c. das aus der Umlenkeinrichtung (9) austretende Beleuchtungslicht (8) versetzt zu dem in die Umlenkeinrichtung (9) eintretenden Beleuchtungslicht (8) verläuft und/oder dass
d. das aus der Umlenkeinrichtung (9) austretende Beleuchtungslicht (8) im Wesentlichen parallel, insbesondere parallel, zu dem in die Umlenkeinrichtung eintretenden Beleuchtungslicht (8) verschoben ist.

3. Dispensiervorrichtung nach Anspruch 1 oder 2, **dadurch gekennzeichnet, dass**
a. das aus der Umlenkeinrichtung (9) austretende Detektionslicht (15) versetzt zu dem in die Umlenkeinrichtung (9) eintretenden Detektionslicht (15) verläuft und/oder dass
b. das aus der Umlenkeinrichtung (9) austretende Detektionslicht (15) im Wesentlichen parallel, insbesondere parallel, zu dem in die Umlenkeinrichtung (9) eintretenden Detektionslicht (15) verschoben ist und/oder dass
c. die Umlenkeinrichtung (9) derart ausgebildet ist, dass das durch die zweite Seite (11) der Umlenkeinrichtung (9) austretende Detektionslicht (15) zu dem durch die erste Seite (10) eintretenden Detektionslicht (15) wenigstens zweimal umgelenkt ist.

4. Dispensiervorrichtung (1) nach einem der Ansprüche 1 bis 3, **dadurch gekennzeichnet, dass**
a. das Prisma (24) eine Grundfläche aufweist, die wenigstens vier Ecken aufweist und/oder dass
b. das Prisma (24) ein schiefes Prisma, insbesondere ein Parallelepiped, vorzugsweise ein Rhomboeder, ist und/oder dass
c. eine erste Seite (10) des Prismas (24), durch die Beleuchtungslicht (8) aus dem Prisma (24) austritt, zu einer zweiten Seite (11) parallel verläuft, durch die Beleuchtungslicht (8) in das Prisma (24) eintritt.

5. Dispensiervorrichtung (1) nach einem der Ansprüche 1 bis 4, **dadurch gekennzeichnet, dass**
a. die optische Erfassungseinrichtung (5) ein Objektiv (12) aufweist und/oder dass
b. die Umlenkeinrichtung (9) im Strahlengang des Detektionslichts (15) und/oder des Beleuchtungslichts (8) zwischen dem Objektiv (12) und dem Dispenser (2) angeordnet ist und/oder dass
c. die Umlenkeinrichtung (9) derart ausgebildet ist, dass sie das Detektionslicht (15), insbesondere direkt, in das Objektiv (12) umlenkt.

6. Dispensiervorrichtung (1) nach einem der Ansprüche 1 bis 5, **dadurch gekennzeichnet, dass**
a. die Umlenkeinrichtung (9) mit dem Dispenser (2) mechanisch verbunden ist und/oder dass
b. die Umlenkeinrichtung (9) an dem Dispenser (2) angebracht ist und/oder dass
c. die Umlenkeinrichtung (9) derart ausgebildet ist, dass sie das Beleuchtungslicht (8) direkt in den Teilbereich (6) umlenkt und/oder dass
d. eine Linse an der zum Dispenser (2) zugewandten Seite der Umlenkeinrichtung (9) angebracht ist oder dass eine plane Seite aufweisende Linse an der zum Dispenser (2) zugewandten Seite der Umlenkeinrichtung (9) angebracht ist.

7. Dispensiervorrichtung (1) nach Anspruch 5 oder 6, **dadurch gekennzeichnet, dass** das Objektiv (12) derart angeordnet ist, dass eine optische Achse (13) des Objektivs (12) quer, insbesondere senkrecht, zu einer Ausgaberichtung (R) der Flüssigkeit aus dem Dispenser (2) verläuft.

8. Dispensiervorrichtung (1) nach einem der Ansprüche 1 bis 7, **gekennzeichnet durch** einen Aktor zum Betätigen des Dispensers (2), wobei
a. der Aktor ein Piezoaktor ist und/oder dass
b. der Aktor und das Objektiv (12) sich bezogen auf den Dispenser (2) gegenüberliegen.

9. Dispensiervorrichtung (1) nach einem der Ansprüche 1 bis 8, **dadurch gekennzeichnet, dass** die optische Erfassungseinrichtung (5) eine Ausleseeinrichtung (14) zum Auslesen des von dem Teilbereich (6) des Dispensers (2) ausgehenden, detektierten Detektionslichts (15) aufweist.

10. Dispensiervorrichtung (1) nach Anspruch 9, **dadurch gekennzeichnet, dass**
a. die optische Erfassungseinrichtung (5) eine Abbildungsvorrichtung (16) aufweist, wobei die Abbildungsvorrichtung (16) basierend auf dem detektierten Detektionslicht (15) eine Abbildung erzeugt und/oder wobei die Abbildungsvorrichtung (16) eine Abbildung von dem Teilbereich (6) des Dispensers (2) erzeugt und/oder dass
b. die optische Erfassungseinrichtung (5) eine Auswerteeinrichtung (17) zum Auswerten des detektierten Detektionslichts (15) aufweist oder dass die optische Erfassungseinrichtung (5) eine Auswerteeinrichtung (17) zum Auswerten des detektierten Detektionslichts (15) aufweist, wobei in der Auswerteeinrichtung (17) ein Algorithmus zum wenigstens teilweisen Beheben von einem Abbildungsfehler hinterlegt ist.

11. Dispensiervorrichtung (1) nach Anspruch 10, **dadurch gekennzeichnet, dass**
a. die Auswerteeinrichtung (17) zum Bestimmen einer optischen Eigenschaft des Teilbereichs (6) des Dispensers (2) das detektierte Detektionslicht (15) auswertet oder dass
b. die Auswerteeinrichtung (17) basierend auf dem detektierten Detektionslicht, insbesondere basierend auf der optischen Eigenschaft des Teilbereichs (6) des Dispensers (2), bestimmt, ob im Teilbereich (6) des Dispensers (2) keine Zelle (1) und/oder kein Partikel angeordnet ist oder ob im Teilbereich (6) des Dispensers (2) genau eine einzige Zelle (3) und/oder ein einziges Partikel angeordnet ist oder ob im Teilbereich (6) des Dispensers (2) mehrere Zellen und/oder mehrere Partikel angeordnet sind.

12. Dispensiervorrichtung (1) nach einem der Ansprüche 1 bis 11, **dadurch gekennzeichnet, dass**
a. die optische Erfassungseinrichtung (5) eine weitere Lichtquelle (18) zum Ausgeben eines Anregungslichts (19) zum Beleuchten des wenigstens einen Teilbereichs (6) des Dispensers (2) und/oder wenigstens eines anderen Teilbereichs des Dispensers (2) aufweist oder dass
b. die optische Erfassungseinrichtung (5) eine weitere Lichtquelle (18) zum Ausgeben eines Anregungslichts (19) zum Beleuchten des wenigstens einen Teilbereichs (6) des Dispensers (2) und/oder wenigstens eines anderen Teilbereichs des Dispensers (2) aufweist und das Anregungslicht (19) und das Beleuchtungslicht (8) wenigstens teilweise einen gemeinsamen Strahlengang besitzen.

13. Dispensiervorrichtung (1) nach Anspruch 12, **dadurch gekennzeichnet, dass**
a. die Ausleseeinrichtung (14) zum Auslesen eines detektierten weiteren Detektionslichts (20) ausgebildet ist und/oder dass
b. die Abbildungsvorrichtung (16) basierend auf einem weiteren Detektionslicht (20) eine weitere Abbildung des Teilbereichs (6) und/oder des anderen Teilbereichs erzeugt und/oder dass
c. die Auswerteeinrichtung (17) zum Bestimmen einer weiteren optischen Eigenschaft des Teilbereichs (6) und/oder des anderen Teilbereichs das detektierte weitere Detektionslicht (20) auswertet und/oder dass
d. die Auswerteeinrichtung (17) basierend auf dem detektierten weiteren Detektionslicht (20) bestimmt, ob im Teilbereich (6) und/oder anderen Teilbereich keine fluoreszierende Zelle (3) und/oder kein fluoreszierendes Partikel angeordnet ist oder ob im Teilbereich (6) und/oder anderen Teilbereich genau eine einzige fluoreszierende Zelle (3) und/oder genau ein einziges fluoreszierendes Partikel angeordnet ist und/oder ob im Teilbereich mehrere Zellen (3) und/oder mehrere Partikel angeordnet sind.

14. Dispensiervorrichtung (1) nach Anspruch 12, **dadurch gekennzeichnet, dass**
a. die optische Erfassungseinrichtung (5) eine weitere Ausleseeinrichtung (21) zum Auslesen eines detektierten weiteren Detektionslichts (20) aufweist und/oder dass
b. die optische Erfassungseinrichtung (5) eine weitere Abbildungsvorrichtung (22) zum Erzeugen einer weiteren Abbildung des Teilbereichs (6) und/oder des anderen Teilbereichs basierend auf dem detektierten weiteren Detektionslicht (20) aufweist.

15. Dispensiervorrichtung (1) nach Anspruch 12 oder 14, **dadurch gekennzeichnet, dass** die optische Erfassungseinrichtung (5) eine weitere Auswerteeinrichtung (23) aufweist, wobei
a. die weitere Auswerteeinrichtung (23) zum Bestimmen einer weiteren optischen Eigenschaft des Teilbereichs (6) und/oder des anderen Teilbereichs das detektierte weitere Detektionslicht (20) auswertet und/oder wobei
b. die weitere Auswerteeinrichtung (23) basierend auf dem weiteren Detektionslicht (20) bestimmt, ob im Teilbereich (6) und/oder anderen Teilbereich keine fluoreszierende Zelle (3) und/oder kein fluoreszierendes Partikel angeordnet ist oder ob im Teilbereich (6) und/oder anderen Teilbereich genau eine einzige fluoreszierende Zelle (3) und/oder genau ein einziges fluoreszierendes Partikel angeordnet ist und/oder ob im Teilbereich mehrere Zellen (3) und/oder mehrere Partikel angeordnet sind..

## Claims

1. A dispensing apparatus (1) having a dispenser (2) for dispensing a liquid (4) containing at least one cell (3) and/or at least one particle and an optical detection device (5) for optically detecting at least a partial region (6) of the dispenser (2), which detection device has a light source (7) for emitting an illumination light (8) for illuminating the partial region (6) and a deflection device (9) for deflecting a detection light (15) emanating from the partial region (6), **characterised in that** the deflection device (9) deflects the detection light (15) at least twice, in particular exactly twice, wherein the deflection device (9) has at least one prism (24) for deflecting the detection light (15) and/or the illumination light (8).

2. The dispensing apparatus (1) according to claim 1, **characterised in that**
a. the deflection device (9) deflects the illumination light (8) at least twice, in particular exactly twice, and/or **in that**
b. the deflection device (9) is designed such that the illumination light (8) emerging through a first side (10) of the deflection device is deflected at least twice to the illumination light (8) entering through a second side (11) and/or **in that**
c. the illumination light (8) emerging from the deflection device (9) runs offset to the illumination light (8) entering the deflection device (9) and/or **in that**
d. the illumination light (8) emerging from the deflection device (9) is displaced substantially parallel, in particular parallel, to the illumination light (8) entering the deflection device.

3. The dispensing apparatus according to claim 1 or 2, **characterised in that**
a. the detection light (15) emerging from the deflection device (9) runs offset to the detection light (15) entering the deflection device (9) and/or **in that**
b. the detection light (15) emerging from the deflection device (9) is displaced substantially parallel, in particular parallel, to the detection light (15) entering the deflection device (9) and/or **in that**
c. the deflection device (9) is designed such that the detection light (15) emerging through the second side (11) of the deflection device (9) is deflected at least twice to the detection light (15) entering through the first side (10).

4. The dispensing apparatus (1) according to any one of claims 1 to 3, **characterised in that**
a. the prism (24) has a base area which has at least four corners and/or **in that**
b. the prism (24) is an oblique prism, in particular a parallelepiped, preferably a rhombohedron, and/or **in that**
c. a first side (10) of the prism (24), through which the illumination light (8) emerges from the prism (24), extends parallel to a second side (11), through which the illumination light (8) enters the prism (24).

5. The dispensing apparatus (1) according to any one of claims 1 to 4, **characterised in that**
a. the optical detection device (5) has an object lens (12) and/or **in that**
b. the deflection device (9) is arranged in the beam path of the detection light (15) and/or of the illumination light (8) between the object lens (12) and the dispenser (2) and/or **in that**
c. the deflection device (9) is designed such that it deflects the detection light (15), in particular directly, into the object lens (12).

6. The dispensing apparatus (1) according to any one of claims 1 to 5, **characterised in that**
a. the deflection device (9) is mechanically connected to the dispenser (2) and/or **in that**
b. the deflection device (9) is attached to the dispenser (2) and/or **in that**
c. the deflection device (9) is designed such that it deflects the illumination light (8) directly into the partial region (6) and/or **in that**
d. a lens is attached to the side of the deflection device (9) facing the dispenser (2) or **in that** a lens having a flat side is attached to the side of the deflection device (9) facing the dispenser (2).

7. The dispensing apparatus (1) according to claim 5 or 6, **characterised in that** the object lens (12) is arranged such that an optical axis (13) of the object lens (12) extends transverse, in particular perpendicular, to a dispensing direction (R) of the liquid from the dispenser (2).

8. The dispensing apparatus (1) according to any one of claims 1 to 7, **characterised by** an actuator for actuating the dispenser (2), wherein
a. the actuator is a piezo actuator and/or in that
b. the actuator and the object lens (12) face each other with respect to the dispenser (2).

9. The dispensing apparatus (1) according to any one of claims 1 to 8, **characterised in that** the optical detection device (5) has a readout device (14) for reading out the detected detection light (15) emanating from the partial region (6) of the dispenser (2).

10. The dispensing apparatus (1) according to claim 9, **characterised in that**
a. the optical detection device (5) has an imaging apparatus (16), wherein the imaging device (16) generates an image on the basis of the detected detection light (15) and/or wherein the imaging device (16) generates an image of the partial region (6) of the dispenser (2) and/or in that
b. the optical detection device (5) has an evaluation device (17) for evaluating the detected detection light (15) or **in that** the optical detection device (5) has an evaluation device (17) for evaluating the detected detection light (15), wherein an algorithm for at least partially rectifying an imaging error is stored in the evaluation device (17).

11. The dispensing apparatus (1) according to claim 10, **characterised in that**
a. the evaluation device (17) for determining an optical property of the partial region (6) of the dispenser (2) evaluates the detected detection light (15) or **in that**
b. on the basis of the detected detection light, in particular on the basis of the optical property of the partial region (6) of the dispenser (2), the evaluation device (17) determines whether no cells (1) and/or no particles are arranged in the partial region (6) of the dispenser (2) or whether exactly one single cell (3) and/or one single particle are/is arranged in the partial region (6) of the dispenser (2) or whether a plurality of cells and/or a plurality of particles are arranged in the partial region (6) of the dispenser (2).

12. The dispensing apparatus (1) according to any one of claims 1 to 11, **characterised in that**
a. the optical detection device (5) has a further light source (18) for emitting an excitation light (19) for illuminating the at least one partial region (6) of the dispenser (2) and/or at least another partial region of the dispenser (2) or **in that**
b. the optical detection device (5) has a further light source (18) for emitting an excitation light (19) for illuminating the at least one partial region (6) of the dispenser (2) and/or at least another partial region of the dispenser (2) and the excitation light (19) and the illumination light (8) at least partially have a common beam path.

13. The dispensing apparatus (1) according to claim 12, **characterised in that**
a. the readout device (14) is designed to read out a detected further detection light (20) and/or **in that**
b. the imaging apparatus (16) generates a further image of the partial region (6) and/or of the other partial region on the basis of a further detection light (20) and/or **in that**
c. the evaluation device (17) for determining a further optical property of the partial region (6) and/or of the other partial region evaluates the detected further detection light (20) and/or in that
d. on the basis of the detected further detection light (20) the evaluation device (17) determines whether no fluorescent cells (3) and/or no fluorescent particles are arranged in the partial region (6) or whether exactly one single fluorescent cell (3) and/or exactly one single fluorescent particle are/is arranged in the partial region (6) and/or the other partial region and/or whether a plurality of cells (3) and/or a plurality of particles are/is arranged in the partial region.

14. The dispensing apparatus (1) according to claim 12, **characterised in that**
a. the optical detection device (5) has a further readout device (21) for reading out a detected further detection light (20) and/or **in that**
b. the optical detection device (5) has a further imaging apparatus (22) for generating a further image of the partial region (6) and/or of the other partial region on the basis of the detected further detection light (20).

15. The dispensing apparatus (1) according to claim 12 or 14, **characterised in that** the optical detection device (5) has a further evaluation device (23), wherein
a. the further evaluation device (23) for determining a further optical property of the partial region (6) and/or the other partial region evaluates the detected further detection light (20) and/or wherein
b. on the basis of the detected further detection light (20) the further evaluation device (23) determines whether no fluorescent cells (3) and/or no fluorescent particles are arranged in the partial region (6) or whether exactly one single fluorescent cell (3) and/or exactly one single fluorescent particle are/is arranged in the partial region (6) and/or the other partial region and/or whether a plurality of cells (3) and/or a plurality of particles are/is arranged in the partial region.

## Revendications

1. Dispositif de distribution (1) avec un distributeur (2) pour distribuer un liquide (4) contenant au moins une cellule (3) et/ou au moins une particule et un dispositif de détection optique (5) pour détecter optiquement au moins une zone partielle (6) du distributeur (2), qui possède une source de lumière (7) pour émettre une lumière d'éclairage (8) pour éclairer la zone partielle (6) et un dispositif de déviation (9) pour dévier une lumière de détection (15) provenant de la zone partielle (6), **caractérisé en ce que** le dispositif de déviation (9) dévie la lumière de détection (15) au moins deux fois, en particulier exactement deux fois, le dispositif de déviation (9) comportant au moins un prisme (24) pour dévier la lumière de détection (15) et/ou la lumière d'éclairage (8).

2. Dispositif de distribution (1) selon la revendication 1, **caractérisé en ce que**
a. le dispositif de déviation (9) dévie la lumière d'éclairage (8) au moins deux fois, en particulier exactement deux fois et/ou que
b. le dispositif de déviation (9) est conçu de telle sorte que la lumière d'éclairage (8) sortant d'un premier côté (10) du dispositif de déviation est déviée au moins deux fois vers la lumière d'éclairage (8) entrant par un deuxième côté (11) et/ou que
c. la lumière d'éclairage (8) sortant du dispositif de déviation (9) est décalée par rapport à la lumière d'éclairage (8) entrant dans le dispositif de déviation (9) et/ou que
d. la lumière d'éclairage (8) sortant du dispositif de déviation (9) est déplacée essentiellement parallèlement, en particulier parallèlement, à la lumière d'éclairage (8) entrant dans le dispositif de déviation.

3. Dispositif de distribution selon la revendication 1 ou 2, **caractérisé en ce que**
a. la lumière de détection (15) sortant du dispositif de déviation (9) est décalée par rapport à la lumière de détection (15) entrant dans le dispositif de déviation (9) et/ou que
b. la lumière de détection (15) sortant du dispositif de déviation (9) est déplacée essentiellement parallèlement, en particulier parallèlement, à la lumière de détection (15) entrant dans le dispositif de déviation (9) et/ou que
c. le dispositif de déviation (9) est conçu de telle sorte que la lumière de détection (15) sortant par le deuxième côté (11) du dispositif de déviation (9) est déviée au moins deux fois vers la lumière de détection (15) entrant par le premier côté (10).

4. Dispositif de distribution (1) selon l'une quelconque des revendications 1 à 3, **caractérisé en ce que**
a. le prisme (24) a une surface de base qui a au moins quatre coins et/ou que
b. le prisme (24) est un prisme oblique, notamment un parallélépipède, de préférence un rhomboèdre et/ou que
c. un premier côté (10) du prisme (24), à travers lequel la lumière d'éclairage (8) sort du prisme (24), est parallèle à un deuxième côté (11) à travers lequel la lumière d'éclairage (8) entre dans le prisme (24).

5. Dispositif de distribution (1) selon l'une quelconque des revendications 1 à 4, **caractérisé en ce que**
a. le dispositif de détection optique (5) comporte un objectif (12) et/ou que
b. le dispositif de déviation (9) est disposé dans le trajet du faisceau de la lumière de détection (15) et/ou de la lumière d'éclairage (8) entre l'objectif (12) et le distributeur (2) et/ou que
c. le dispositif de déviation (9) est conçu de telle sorte qu'il dévie la lumière de détection (15), en particulier directement, dans l'objectif (12).

6. Dispositif de distribution (1) selon l'une quelconque des revendications 1 à 5, **caractérisé en ce que**
a. le dispositif de déviation (9) est relié mécaniquement au distributeur (2) et/ou que
b. le dispositif de déviation (9) est fixé au distributeur (2) et/ou que
c. le dispositif de déviation (9) est conçu de telle sorte qu'il dévie la lumière d'éclairage (8) directement dans la zone partielle (6) et/ou que
d. une lentille est fixée sur le côté du dispositif de déviation (9) faisant face au distributeur (2) ou qu'une lentille présentant un côté plat est fixée sur le côté du dispositif de déviation (9) faisant face au distributeur (2).

7. Dispositif de distribution (1) selon la revendication 5 ou 6, **caractérisé en ce que** l'objectif (12) est agencé de sorte qu'un axe optique (13) de l'objectif (12) s'étende transversalement, notamment perpendiculairement, à une direction de distribution (R) du liquide depuis le distributeur (2).

8. Dispositif de distribution (1) selon l'une quelconque des revendications 1 à 7, **caractérisé par** un actionneur pour actionner le distributeur (2), dans lequel
a. l'actionneur est un actionneur piézoélectrique et/ou que
b. l'actionneur et l'objectif (12) se font face par rapport au distributeur (2).

9. Dispositif de distribution (1) selon l'une quelconque des revendications 1 à 8, **caractérisé en ce que** le dispositif de détection optique (5) possède un dispositif de lecture (14) pour lire la lumière de détection (15) détectée, émanant de la zone partielle (6) du distributeur (2).

10. Dispositif de distribution (1) selon la revendication 9, **caractérisé en ce que**
a. le dispositif de détection optique (5) possède un dispositif d'imagerie (16), le dispositif d'imagerie (16) générant une image sur la base de la lumière de détection (15) détectée et/ou le dispositif d'imagerie (16) générant une image de la zone partielle (6) du distributeur (2) et/ou que
b. le dispositif de détection optique (5) possède un dispositif d'évaluation (17) pour évaluer la lumière de détection (15) détectée ou que le dispositif de détection optique (5) possède un dispositif d'évaluation (17) pour évaluer la lumière de détection (15) détectée, dans lequel dans le dispositif d'évaluation (17), un algorithme est stocké pour corriger au moins partiellement une erreur d'imagerie.

11. Dispositif de distribution (1) selon la revendication 10, **caractérisé en ce que**
a. le dispositif d'évaluation (17) évalue la lumière de détection (15) détectée pour déterminer une propriété optique de la zone partielle (6) du distributeur (2) ou que
b. le dispositif d'évaluation (17) détermine sur la base de la lumière de détection détectée, en particulier sur la base de la propriété optique de la zone partielle (6) du distributeur (2), s'il n'y a aucune cellule (1) et/ou aucune particule dans la zone partielle (6) du distributeur (2) ou si exactement une seule cellule (3) et/ou une seule particule est disposée dans la zone partielle (6) du distributeur (2) ou si plusieurs cellules et/ou plusieurs particules sont disposées dans la zone partielle (6) du distributeur (2).

12. Dispositif de distribution (1) selon l'une quelconque des revendications 1 à 11, **caractérisé en ce que**
a. le dispositif de détection optique (5) possède une autre source de lumière (18) pour émettre une lumière d'excitation (19) pour éclairer la au moins une zone partielle (6) du distributeur (2) et/ou au moins une autre zone partielle du distributeur (2) ou que
b. le dispositif de détection optique (5) possède une autre source de lumière (18) pour émettre une lumière d'excitation (19) pour éclairer la au moins une zone partielle (6) du distributeur (2) et/ou au moins une autre zone partielle du distributeur (2) et la lumière d'excitation (19) et la lumière d'éclairage (8) ont au moins partiellement un trajet de faisceau commun.

13. Dispositif de distribution (1) selon la revendication 12, **caractérisé en ce que**
a. le dispositif de lecture (14) est conçu pour lire une autre lumière de détection (20) détectée et/ou que
b. le dispositif d'imagerie (16) génère une autre image de la zone partielle (6) et/ou de l'autre zone partielle sur la base d'une autre lumière de détection (20) et/ou que
c. le dispositif d'évaluation (17) évalue l'autre lumière de détection (20) détectée pour déterminer une autre propriété optique de la zone partielle (6) et/ou de l'autre zone partielle et/ou que
d. le dispositif d'évaluation (17) détermine sur la base de l'autre lumière de détection (20) détectée si aucune cellule fluorescente (3) et/ou aucune particule fluorescente n'est disposée dans la zone partielle (6) et/ou l'autre zone partielle ou si dans la zone partielle (6) et/ou dans l'autre zone partielle, exactement une seule cellule fluorescente (3) et/ou exactement une seule particule fluorescente est disposée et/ou si dans la zone partielle, plusieurs cellules (3) et/ou plusieurs particules sont disposées.

14. Dispositif de distribution (1) selon la revendication 12, **caractérisé en ce que**
a. le dispositif de détection optique (5) possède un autre dispositif de lecture (21) pour lire une autre lumière de détection (20) détectée et/ou que
b. le dispositif de détection optique (5) possède un autre dispositif d'imagerie (22) pour générer une autre image de la zone partielle (6) et/ou de l'autre zone partielle sur la base de l'autre lumière de détection (20) détectée.

15. Dispositif de distribution (1) selon la revendication 12 ou 14, **caractérisé en ce que** le dispositif de détection optique (5) possède un autre dispositif d'évaluation (23), dans lequel
a. l'autre dispositif d'évaluation (23) évalue l'autre lumière de détection (20) détectée pour déterminer une autre propriété optique de la zone partielle (6) et/ou de l'autre zone partielle et/ou dans lequel
b. l'autre dispositif d'évaluation (23) détermine sur la base de l'autre lumière de détection (20) si aucune cellule fluorescente (3) et/ou aucune particule fluorescente n'est disposée dans la zone partielle (6) et/ou l'autre zone partielle ou si dans la zone partielle (6) et/ou dans l'autre zone partielle, exactement une seule cellule fluorescente (3) et/ou exactement une seule particule fluorescente est disposée et/ou si dans la zone partielle, plusieurs cellules (3) et/ou plusieurs particules sont disposées.
